# EUROPEAN PATENT APPLICATION

(11) **EP 4 559 455 A1**
(43) Date of publication of application: **28.05.2025**
(21) Application number: 23842355.2
(22) Date of filing: 19.07.2023
(51) Int. Cl.: A61K 9/127, A61K 31/136, A61P 25/00

(54) **USE OF MITOXANTRONE HYDROCHLORIDE LIPOSOME**

(30) Priority: 20.07.2022 CN 202210856534; 02.08.2022 CN 202210924224
(71) Applicant: CSPC Zhongqi Pharmaceutical Technology (Shijiazhuang) Co., Ltd., Shijiazhuang, Hebei 050035 (CN)
(72) Inventor: LI, Chunlei, Shijiazhuang, Hebei 050035 (CN); LIU, Jiaqi, Shijiazhuang, Hebei 050035 (CN); LI, Yanhui, Shijiazhuang, Hebei 050035 (CN); YU, Yulou, Shijiazhuang, Hebei 050035 (CN); YANG, Xiugao, Shijiazhuang, Hebei 050035 (CN); LI, Mengmeng, Shijiazhuang, Hebei 050035 (CN); WU, Guohua, Shijiazhuang, Hebei 050035 (CN); DU, Yanling, Shijiazhuang, Hebei 050035 (CN); ZHOU, Meng, Shijiazhuang, Hebei 050035 (CN); WANG, Shixia, Shijiazhuang, Hebei 050035 (CN); LI, Chao, Shijiazhuang, Hebei 050035 (CN)
(74) Representative: Gill Jennings & Every LLP
(86) International application number: PCT/CN2023/108139
(87) International publication number: WO 2024/017293

(57) **Abstract**

The present application provides use of mitoxantrone hydrochloride liposome in treatment of neuromyelitis optica spectrum disorders. The present application further provides a method for treating neuromyelitis optica spectrum disorders in an individual. The method comprises administering to the individual a therapeutically effective amount of mitoxantrone hydrochloride liposome or a pharmaceutical composition comprising mitoxantrone hydrochloride liposome. Animal and clinical test results show that mitoxantrone hydrochloride liposome is safe and effective in treatment of neuromyelitis optica spectrum disorders.

## Description

### RELATED APPLICATION

This application claims priority to Chinese Patent Application No. 202210856534.9, filed on July 20, 2022, and Chinese Patent Application No. 202210924224.6, filed on August 2, 2022, the entire contents of which are incorporated herein by reference in their entirety for all purposes.

### TECHNICAL FIELD

The present application relates to the field of medicine, in particular to use of mitoxantrone hydrochloride liposomes, e.g. in the treatment of neuromyelitis optica spectrum disorders.

### BACKGROUND

Neuromyelitis optica spectrum disorder (NMOSD) is an autoimmune-mediated inflammatory demyelinating disease of the central nervous system, characterized by simultaneous or sequential involvement of the optic nerve and spinal cord. Its pathogenesis is mainly related to aquaporin-4 (AQP4) antibody, mediated by autoantibodies, dominated by humoral immunity, and involved by various immune cells and factors. NMOSD is found in all ages, predominantly in young adults, with an average onset age of about 40 years old. Serum AQP4-IgG is positive in most patients. Among AQP4-IgG-positive patients, the ratio of females to males is up to (4.7~11): 1. Clinically, severe optic neuritis and longitudinally extending long-segment transverse myelitis are the main clinical features, often accompanied by NMO-characteristic brain lesions (in hypothalamus, corpus callosum, periventricular or brainstem) or systemic autoimmune diseases. NMOSD is a highly recurrent and disabling disease, and more than 90% patients have a multiphase course, 40%-60% of them relapsed within 1 year, and about 90% relapsed within 3 years. About 50% of patients with natural course of the disease have left severe visual or motor dysfunction within 5 to 10 years (Neuroimmunology Branch of Chinese Society for Immunology, Dehui Huang, Weiping Wu et al., Chinese guidelines for the diagnosis and treatment of neuromyelitis optica spectrum disorders (2021 version), Chinese Journal of Neuroimmunology and Neurology, Nov. 2021, Vol. 28, No. 6).

Evidence from pathogenesis, imaging, and clinical manifestations suggests that NMOSD is an independent disease entity different from multiple sclerosis (MS), and its treatment regimen is also different from that of MS. Some drugs for treating MS may aggravate the deterioration of NMOSD (Wei Ji, Limei Wang, Song Tan, Progress in the identification, diagnosis and treatment of neuromyelitis optica and multiple sclerosis by laboratory indexes, Chinese Journal of Practical Nervous Diseases, June 2016, Vol. 19, No. 10).

Currently, the commonly used clinical treatment of NMOSD is divided into acute attack treatment, sequential treatment (treatment of relapse prevention), symptomatic treatment and rehabilitation treatment. The purpose of acute phase treatment is to reduce irreversible damage to the nerve, to promote the recovery of its function, and to reduce mortality rate. In acute phase treatment, high-dose hormone shock therapy, plasma exchange or immunoadsorption, and intravenous injection of human immunoglobulins and the like are often used. The purpose of sequential treatment is mainly to decrease the recurrence rate and reduce the severity of disabilities in patients. Commonly used clinical drugs for sequential treatment include monoclonal antibodies and immunosuppressants. First-line drugs include satralizumab, inebilizumab, rituximab, azathioprine, and mycophenolate mofetil. Second-line drugs include tacrolimus, cyclosporin A, cyclophosphamide, methotrexate, and mitoxantrone. (Neuroimmunology Branch of Chinese Society for Immunology, Dehui Huang, Weiping Wu et al., Chinese guidelines for the diagnosis and treatment of neuromyelitis optica spectrum disorders (2021 version), Chinese Journal of Neuroimmunology and Neurology, Nov. 2021, Vol. 28, No. 6).

In view of the highly recurrent and highly disabling characteristics of NMOSD, a large number of patients with NMOSD still cannot be effectively treated, and their diseases are not effectively controlled, leaving serious physical disability, and causing heavy disease burden on the families and society. Therefore, there is an urgent need for early diagnosis of NMOSD, and drugs for effectively treating NMOSD, and preventing recurrence of NMOSD.

Mitoxantrone hydrochloride is a kind of anthraquinone chemotherapeutic agent. The indications for conventional mitoxantrone hydrochloride injections approved by the FDA are multiple sclerosis, prostate cancer and acute myeloid leukemia. The injections are approved in China for patients with lymphoma, leukemia and breast cancer and the like. When used alone, the recommended dose for adults is 12-14 mg/m², once every 3-4 weeks; or 4-8 mg/m², once a day for 3-5 days at intervals of 2-3 weeks. When used in combination, the dose is 5-10 mg/m² once. The main adverse reactions of mitoxantrone as one of anthracyclines are mainly cardiotoxicity, bone marrow suppression and gastrointestinal reactions and the like.

Among them, bone marrow suppression and gastrointestinal reactions can be solved by the administration of appropriate drugs. However, cardiotoxicity tends to have serious consequences, which is the most serious adverse reaction of anthracyclines. Clinical research and practical observations have shown that the cardiotoxicity caused by anthracyclines is mostly progressive and irreversible. Especially, the heart damage is proned to be caused by the first use of anthracyclines. Chronic dose cumulative limiting toxicity-cardiotoxicity is a common concern of clinicians. Therefore, although breast cancer has been approved as its indication, mitoxantrone has not been recommended as a therapy for breast cancer by authoritative clinical medication guidelines.

Different tumors show different drug sensitivity. Previous studies have shown that the dosage regimen of the same drug may be different in the treatment of different indications. For example, Doxil (doxorubicin hydrochloride liposome) has been approved by the FDA for the following three indications: (1) ovarian cancer: recommended dosage of 50 mg/m², intravenously administrated every 4 weeks; (2) Kaposi's sarcoma: recommended dosage of 20 mg/m², intravenously administrated every 3 weeks; and (3) multiple myeloma: recommended dosage of 30 mg/m², intravenously administrated on the fourth day after the administration of bortezomib. Another example is Abraxane (paclitaxel for injection [Albumin Bound]) has also been approved by the FDA for the following three indications: (1) metastatic breast cancer: recommended dosage of 260 mg/m², intravenously dripped for 30 min, administrated once every 3 weeks; (2) non-small cell lung cancer: recommended dosage of 100 mg/m², intravenously dripped for 30 min, with a course of treatment every 21 days, administrated on days 1, 8 and 15, respectively; on the first day, carboplatin given immediately after the administration of paclitaxel for injection [Albumin Bound]), administrated once every 21 days; (3) pancreatic cancer: recommended dosage of 125 mg/m², intravenously dripped for 30-40 min, with a treatment period every 28 days, administrated on days 1, 8 and 15, respectively; gemcitabine given immediately after each administration of paclitaxel for injection [Albumin Bound]). Another example is AmBisome (Amphotericin B Liposome for Injection), the initial dose of which for the treatment of the following indications are as follows: (1) empirical treatment: recommended dosage of 3mg/kg/day; (2) systemic fungal infection (Aspergillus, Candida, or Cryptococcus): recommended dosage of 3~5mg/kg/day; (3) cryptococcal meningitis in HIV-infected patients: recommended dosage of 6mg/kg/day (Days 1 to 5), and 3mg/kg/day (Days 4 and 21); (4) patients with immunocompromised visceral leishmaniasis: 4mg/kg/day (Days 1 to 5), and 4mg/kg/day (Days 10, 17, 24, 31, and 38). Dosage and administration data are personalized according to actual condition of patients, so as to achieve maximum pharmacological efficacy and minimum toxicity or adverse effects. It can be seen that there are differences in the safe and effective dosages of the same drug for the treatment of different indications. The dosage and administration data should be personalized according to specific diseases and the actual condition of patients in order to achieve maximum pharmacological efficacy and minimal toxicity or adverse effects, and to achieve safe and effective treatment of diseases. The absorption, distribution, and metabolism of mitoxantrone liposome (a special dosage form different from conventional injections) after entering the body are very complicated, and it is difficult to simply deduce from one indication to another for the treatment of different indications, especially for the treatment of different tumors.

### SUMMARY OF THE INVENTION

The present application provides use of mitoxantrone hydrochloride liposome in the treatment of neuromyelitis optica spectrum disorders. The inventors of the present application have surprisingly found in the study that the mitoxantrone hydrochloride liposome has a good therapeutic effect on neuromyelitis optica spectrum disorders and can be used for preparing related medicaments.

In a first aspect, the present application provides use of mitoxantrone hydrochloride liposome in the manufacture of a medicament for treating neuromyelitis optica spectrum disorders.

In a second aspect, the present application provides a method for treating neuromyelitis optica spectrum disorders in an individual, comprising administering to the individual a therapeutically effective amount of mitoxantrone hydrochloride liposome or a pharmaceutical composition containing a therapeutically effective amount of mitoxantrone hydrochloride liposome.

In a third aspect, the present application provides mitoxantrone hydrochloride liposome or a pharmaceutical composition comprising mitoxantrone hydrochloride liposome for use in the treatment of neuromyelitis optica spectrum disorders in an individual.

In some embodiments, the neuromyelitis optica spectrum disorders described in the above first to third aspects are highly recurrent neuromyelitis optica spectrum disorders. In some embodiments, the highly recurrent neuromyelitis optica spectrum disorders are highly recurrent neuromyelitis optica spectrum disorders for which the treatment with other drugs failed.

In some embodiments, for the highly recurrent neuromyelitis optica spectrum disorders for which the treatment with other drugs failed, the other drugs are selected from the group consisting of first-line, second-line or above drugs for the neuromyelitis optica spectrum disorders, especially those approved by the drug administration in China or abroad (e.g., the United States, the European Union, Japan, and Korea) for the treatment of neuromyelitis optica spectrum disorders, including but not limited to glucocorticoids, monoclonal antibody drugs, and immunosuppressants approved by the FDA.

In some embodiments, the mitoxantrone hydrochloride liposome is used as the sole active ingredient for the treatment of neuromyelitis optica spectrum disorders or as the sole active ingredient to prepare a medicament or a pharmaceutical composition for the treatment of neuromyelitis optica spectrum disorders.

In some embodiments, the pharmaceutical composition or medicament comprising mitoxantrone hydrochloride liposome described in the above first to third aspects is an injectable formulation, including a liquid injection, a powder for injection, or a tablet for injection. In a preferred embodiment, the pharmaceutical composition or medicament containing mitoxantrone hydrochloride liposome is a liquid injection.

In some embodiments, when the pharmaceutical composition or medicament comprising mitoxantrone hydrochloride liposome is a liquid injection, the content of mitoxantrone in the medicament or pharmaceutical composition, calculated as mitoxantrone, is 0.5-5 mg/ml based on mitoxantrone. In a preferred embodiment, the content of mitoxantrone in the medicament is 1-2 mg/mL. In a more preferred embodiment, the content of mitoxantrone in the medicament or pharmaceutical composition is 1 mg/mL.

In some embodiments, the therapeutically effective amount in the second aspect above is 4-30 mg/m², for example, 8-20 mg/m² or 12-30 mg/m², or a dosage in the range between any two of the above dosages, for example, 8 mg/m², 12 mg/m², etc. calculated as mitoxantrone.

In some embodiments, the mitoxantrone hydrochloride liposome, the pharmaceutical composition or medicament comprising mitoxantrone hydrochloride liposome of the present application is administered intravenously to an individual in need thereof.

In some embodiments, the mitoxantrone hydrochloride liposome, the pharmaceutical composition or medicament comprising mitoxantrone hydrochloride liposome of the present application is administered once every 12 weeks.

In some embodiments, the liposome is used alone to treat neuromyelitis optica spectrum disorders in an individual. In some embodiments, the therapeutically effective amount (calculated as mitoxantrone) of the liposome is 4-30 mg/m², for example, 8-20 mg/m² or 12-30 mg/m², or a dosage in the range between any two of the above dosages, for example, 8 mg/m² or 12 mg/m². In some embodiments, the liposome is administered intravenously.

In some embodiments, the liposome is administered once every 12 weeks.

In the context of the present application, the dosage is calculated based on mitoxantrone, unless otherwise specified.

In the context of the present application, the mitoxantrone hydrochloride liposome can be prepared by methods known in the art and can be one prepared by any of methods disclosed in the prior art, for example prepared by the method disclosed in WO2008/080367 A1.

In some embodiments, the medicament, mitoxantrone hydrochloride liposome, or pharmaceutical composition comprising mitoxantrone hydrochloride liposome described in the above first to third aspects has one or more of the following properties.
(i) The mitoxantrone hydrochloride liposome has a particle size of about 30-80 nm, for example about 35-75 nm, about 40-70 nm, about 40-60 nm, or about 60 nm.
(ii) Mitoxantrone hydrochloride forms a poorly soluble precipitate with multivalent counterions (such as sulfate radicals, citrate radicals or phosphate radicals) in the liposome.
(iii) The phospholipid bilayer in the mitoxantrone hydrochloride liposome comprises phospholipids with a phase transition temperature (Tm) higher than body temperature, such that the phase transition temperature of the liposome is higher than body temperature; and for example, the phospholipids are selected from the group consisting of hydrogenated soybean lecithin, phosphatidylcholine, hydrogenated egg yolk lecithin, bispalmitate lecithin, bisstearate lecithin, or any combination thereof.
(iv) The phospholipid bilayer in the mitoxantrone hydrochloride liposome comprises hydrogenated soybean lecithin, cholesterol and polyethylene glycol 2000 modified distearoyl phosphatidylethanolamine (DSPE-PEG2000).
(iiv) The phospholipid bilayer in the mitoxantrone hydrochloride liposome comprises hydrogenated soybean lecithin, cholesterol and polyethylene glycol 2000 modified distearoyl phosphatidylethanolamine in a mass ratio of about 3:1:1; mitoxantrone hydrochloride forms a poorly soluble precipitate with multivalent acid group anions in the liposome; and the particle size of the mitoxantrone hydrochloride liposome in the medicament is about 60 nm.
(iiiv) The mitoxantrone hydrochloride liposome is the mitoxantrone hydrochloride liposome of National Medicine Permission Number H20220001.

In some embodiments, the mitoxantrone hydrochloride liposome described herein has a particle size of about 30-80 nm, for example, any numberical value between 30-80 nm. In some embodiments, the particle size is about 35-75 nm, preferably 40-70 nm, and more preferably 40-60 nm, for example 60 nm.

In some embodiments, the mitoxantrone hydrochloride liposome described herein comprises the active ingredient mitoxantrone, and a phospholipid bilayer.

In some embodiments, the active ingredient mitoxantrone can form a poorly soluble precipitate with the multivalent counterions in the liposome. In some embodiments, the counterions are sulfate radicals, citrate radicals, or phosphate radicals.

In some embodiments, the above phospholipid bilayer comprises phospholipids with a phase transition temperature (Tm) higher than body temperature, such that the phase transition temperature of the liposome is higher than body temperature. In some embodiments, the phospholipids having a Tm above body temperature are phosphatidylcholine, hydrogenated soybean lecithin, hydrogenated egg yolk lecithin, bispalmitate lecithin, bisstearate lecithin, or any combination thereof.

In some embodiments, the phospholipid bilayer comprises hydrogenated soybean lecithin, cholesterol, and polyethylene glycol 2000 modified distearoyl phosphatidylethanolamine. In some embodiments, the mass ratio of hydrogenated soybean lecithin, cholesterol, and polyethylene glycol 2000 modified distearoyl phosphatidylethanolamine in the phospholipid bilayer is 3:1:1.

In some embodiments, the mitoxantrone hydrochloride liposome described herein has a particle size of about 60 nm and the counterion in the liposome is a sulfate ion.

In some embodiments, the phospholipid bilayer of mitoxantrone hydrochloride liposome described herein comprises hydrogenated soybean lecithin, cholesterol, and polyethylene glycol 2000 modified distearoylphosphatidylethanolamine in a mass ratio of 3:1:1, the liposome has a particle size of about 40-60 nm, and the counterion in the liposome is sulfate ion. In some embodiments, the weight ratio of HSPC: Chol: DSPE-PEG2000: mitoxantrone in the mitoxantrone hydrochloride liposome described herein is 9.58:3.19:3.19:1.

In the context of the present application, the method for preparing the mitoxantrone hydrochloride liposome is as follows. HSPC (hydrogenated soybean lecithin), Chol (cholesterol) and DSPE-PEG2000 (polyethylene glycol 2000 modified distearoyl phosphatidylethanolamine) were weighed according to the mass ratio of 3:1:1 and dissolved in 95% ethanol to obtain a clear solution. The ethanol solution of phospholipids was mixed with a 300 mM ammonium sulfate solution and shaken and hydrated at 60-65°C for 1 h to obtain a heterogeneous multi-compartment liposome. The particle size of the liposome was then reduced using a microfluidic device. The resulting sample was diluted 200-fold with 0.9% NaCl solution and then detected with NanoZS. The average particle size of the particles was about 60 nm and the main peak was concentrated between 40-60 nm. The ammonium sulfate of the external phase of the blank liposome was then removed by an ultrafiltration device and the external phase was replaced with 290 mM sucrose and 10 mM glycine so as to form a transmembrane ammonium sulfate gradient. A solution of mitoxantrone hydrochloride (10 mg/mL) was added to the blank liposome in a ratio of lipid to drug of 16:1 and the drug was loaded at 60-65°C. After incubation for about 1 h, the encapsulation efficiency can be demonstrated to be about 100% by using gel exclusion chromatography. The weight ratio of HSPC: Chol: DSPE-PEG2000: mitoxantrone was 9.58:3.19:3.19:1, and the osmotic pressure of the sucrose glycine solution was close to the physiological value.

It will be appreciated that many technical details and parameters in the above exemplary preparation methods can be adjusted and determined by those skilled in the art within a reasonable range. For example, the amino acid species that can be replaced by glycine in the external phase used to form transmembrane ammonium sulfate gradient include, but are not limited to, histidine, asparagine, glutamic acid, leucine, proline, and alanine. For another example, the mass ratio of HSPC, Chol and DSPE-PEG2000 can be adjusted appropriately. For example, for the parameter of lipid-drug ratio in the preparation of a specific liposomal pharmaceutical formulation, those skilled in the art can design, test, and ultimately derive a suitable lipid-drug ratio to maximize drug loading while reducing drug leakage. For the mitoxantrone hydrochloride liposomal formulation of the present application, the lipid-drug ratio that can be used is in a wide range. For example, the lipid-drug ratio can be as low as 2:1 or as high as 30:1, 40:1, or 50: 1, and a more suitable lipid-drug ratio can be about (15-20): 1, e.g., about 15: 1, 16: 1, 17: 1, 18: 1, 19: 1, or 20: 1. Therefore, the several advantageous properties of the mitoxantrone hydrochloride liposomal formulation described above are more important, and methodologies for achieving these properties are diverse.

As used herein, the term "individual" refers to mammals, such as humans, but may also be other mammals, such as livestock or laboratory animals.

As used herein, the term "treatment/treating" means administration of the mitoxantrone hydrochloride liposome or formulation in the present application to ameliorate or eliminate a disease or one or more symptoms associated with the disease, and includes: (i) inhibiting the disease or disease state, i.e., suppressing its progression; (ii) alleviating the disease or disease state, i.e. causing the disease or disease state to resolve.

The term "therapeutically effective amount" means an amount of the mitoxantrone hydrochloride liposome of the present application that (i) treats a particular disease, condition or disorder, or (ii) alleviates, ameliorates or eliminates one or more symptoms of the particular disease, condition or disorder. The amount of the mitoxantrone hydrochloride liposome of the present application that constitutes the "therapeutically effective amount" varies depending on the compound, the disease state and its severity, the mode of administration, and the age of the mammal to be treated, but can be routinely determined by those skilled in the art based on their own knowledge and the disclosure herein.

In some embodiments, the medicament, mitoxantrone hydrochloride liposome, or pharmaceutical composition comprising mitoxantrone hydrochloride liposome described in the above first to third aspects, such as mitoxantrone hydrochloride liposome injection, has one or more of the following beneficial effects in the treatment of neuromyelitis optica spectrum disorders: safety and tolerance, low toxicity and side effects, improved therapeutic effectiveness, delayed recurrence of neuromyelitis optica spectrum disorders, and the like.

### BRIEF DESCRIPTION OF THE DRAWINGS

FIG. 1 shows an analysis on the weight of animals suffering from neuromyelitis optica. Data is expressed by mean ± SEM. ***P<0.001.
FIG. 2 shows a representative image of AQP4 immunofluorescence staining. Bar: 200 µm.
FIG. 3 shows the statistics of AQP4 positive cell numbers. The data is expressed by mean ± SEM. *P<0.05, **P<0.01, ***P<0.001.

### DETAILED DESCRIPTION

The technical solution of the present application will now be described in further details below in connection with specific examples. It should be understood that the following examples are merely illustrative and explanatory of the present application, and should not be construed as limiting the protection scope of the application. All techniques implemented based on the above contents of this application are intended to be encompassed within the protection scope of the present application.

Unless otherwise stated, the starting materials and reagents used in the following examples are commercially available or can be prepared by known methods.

### Example 1: Improvement effect of mitoxantrone liposome on NMOSD model induced by combination of AQP4 antibody from clinical patients/complement.

It was reported in the literatures that NMOSD model can be induced by injecting the AQP4 antibody from clinical patients diagnosed with NMOSD and human complement directly into the cerebral hemisphere or ventricle of mice (Saadoun S, Waters P, Bell BA, et al. Intra-cerebral injection of neuromyelitis optica immunoglobulin G and human complement produces neuromyelitis optica lesions in mice. Brain. 2010, 133:349-61). Therefore, in this example, the above modelling method was selected to study the therapeutic effect of mitoxantrone liposome on NMOSD.

Experimental animals: CD-1 male mice, 8 weeks of age, purchased from Jinan Pengyue Laboratory Animal Breeding Co., Ltd.

Experimental drug: 1.0 mg/ml mitoxantrone hydrochloride liposome injection (test article).

Experimental method: Firstly, the AQP4-IgG antibody from the blood samples of clinical patients with NMOSD and normal human complement serum were extracted, purified and verified. Thereafter, twenty-seven 8-week-old CD-1 male mice were selected. After one week of adaptation, the mice were randomly divided into following 3 groups based on their body weights: a blank group, a model group and a test article group (15 mg/kg mitoxantrone liposome). Each group consisted of 9 animals.

The lateral ventricles of each mouse in the model and test article groups were injected with 2 µl APQ4 antibody (18mg/ml) plus 1 µl human complement serum at a rate of 0.3 µl/min on days 1, 3 and 5 to induce the NMOSD model.

The blank group was a sham group, and the animal modeling process was the same as that of the model group and the test article group, but without injection of any antibodies and complement.

The animals in the test article group were treated with a single intravenous injection of 15 mg/kg mitoxantrone hydrochloride liposome injection on the first day of modeling.

During the experiment, animals were monitored for health status, and animal body weights were recorded. Three animals in each group were taken at one time and were euthanized with carbon dioxide at day 7, day 11 and day 21 after completion of the process of modelling, and then brain tissues were collected and the pathological changes of brain tissues in mice were evaluated by AQP4, GFAP and LFB staining. With respect to pathological collection sites, the stained area was centered on the injection site, and 30 slices were sectioned to further explore the extent of lesion.

The steps required for the immunohistochemistry procedure and antibody information were as follows:
1. Perfusion-Fixation-Precipitation in Sugar-Slicing
   1) Mice were anesthetized with isoflurane.
   2) The abdominal cavity and chest cavity of the mice were open with direct shear to expose the heart. The blood was firstly perfused and flushed with normal saline, and then perfused with paraformaldehyde for initial fixation.
   3) The head was cut off, and the skull was carefully opened with forceps. The brain tissue was taken out and placed into paraformaldehyde solution for 24 h fixation.
   4) The brain tissue was taken out the next day and put into 20% sucrose solution for 24 h.
   5) The brain tissue was taken out on the third day and put into 30% sucrose solution for 24 h.
   6) The brain tissue was taken out on the fourth day and put into 35% sucrose solution for 24 h. (Appropriate increase in time or concentration depending on the precipitation of brain tissue in sugar)
   7) Brain tissue was taken out, embedded with an OCT embedding agent, and sliced into16 µm brain slices by a frozen section machine.
2. Immunofluorescence staining (AQP4, GFAP)
   1) The slice was rewarmed for 30 min.
   2) Blocking: 10% serum+0.3% TritonX-100 [about 50µl per slice] were added and kept at room temperature for 1 h.
   3) The slice was spinned, and a primary antibody (diluted with PBS) was added to the slice and kept at 4°C overnight.
   4) The slice was rewarmed for 30 min.
   5) The primary antibody was washed off with PBS for 5min×3 times.
   6) A secondary antibody (diluted with PBS) was added to the slice in the dark and kept at room temperature for 2 h.
   7) The secondary antibody was washed off with PBS for 5min×3 times.
   8) DAPI was added to the slice and kept at the room temperature for 10min.
   9) DAPI was washed off for 5min×3 times.
   10) 70% glycerol was added to mount the slice, and bubbles should be avoided.
3. Antibody information:
   3.1. Primary antibody
      1) AQP4: purchased from Abclonal, Article No. A2887, Batch No. 0073660402;
      2) GFAP: purchased from Huaan Biology, Article No. EM140707, Batch No. HO0604;
   3.2. Secondary antibody
      1) Goat Pab to Rabbit 594, purchased from abcam, Article No. ab150080, Batch No.GR3439696-1;
      2) Goat Pab to mouse 488, purchased from abcam, Article No. ab150077, Batch No. GR3403178-2
4. Luxol fast blue stain (LFB) staining

The kit is purchased from Solarbio, Article No. G3242, Batch No. 20220422. The staining process was carried out according to the kit instructions.

### Experimental results

In this study, CD-1 mice were selected to establish a neuromyelitis optica disorder model by injecting the AQP4 antibody from clinically diagnosed patients and the complement into the lateral ventricle of the mice in a manner of stereotactic localization of the brain, and relevant histological tests were performed to evaluate the effect of the compound to be tested.

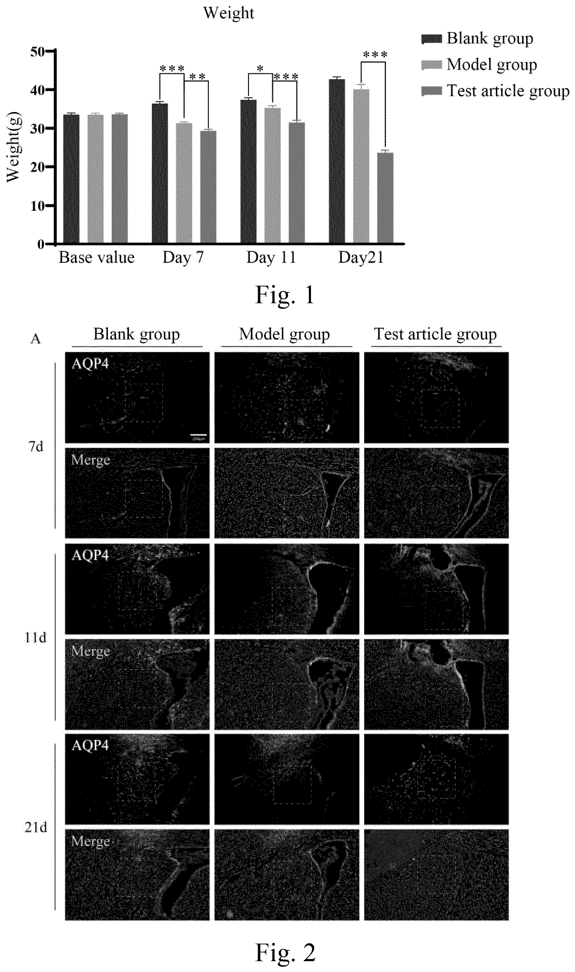

The results of animal body weight analysis were shown in Figure 1 and Table 1. Compared to the model group, the animals in the test article group had decreased body weight from day 7 to day 21 after modelling and drug administration, and were in good condition and no animal death was caused by drug toxicity.

**Table 1**

| Time point | Group | Mean±SEM | P value |
|---|---|---|---|
| Base value | Blank group | 33.5478±0.42584 | 0.971 |
| | Model group | 33.5278±0.42153 | / |
| | Test article group | 33.6422±0.30372 | 0.836 |
| Day 7 | Blank group | 36.4411±0.49304*** | 0.000 |
| | Model group | 31.3067±0.31885 | / |
| | Test article group | 29.3256±0.40197** | 0.002 |
| Day 11 | Blank group | 37.3783±0.56189* | 0.022 |
| | Model group | 35.2517±0.60161 | / |
| | Test article group | 31.47±0.60584*** | 0.000 |
| Day 21 | Blank group | 42.7267±0.64276 | 0.094 |
| | Model group | 40.0967±1.31607 | / |
| | Test article group | 23.6267±0.68919*** | 0.000 |

| | | | |
|---|---|---|---|
| Note: Mean±SEM, n=3~9; Compared to model group: *p<0.05, **p<0.01, ***p<0.001. | | | |

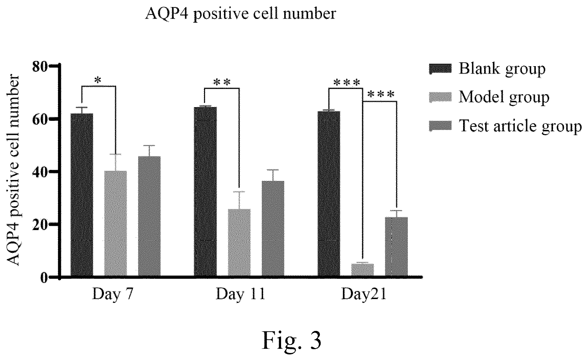

The representative image of AQP4 immunofluorescence staining and the statistical result of AQP4 positive cell numbers were shown in Figures 2-3 and Table 2. On day 7 after modeling, the number of AQP4 positive cells in the brain striatum of the animals in the model group was significantly decreased compared to the blank and test article groups, and this decrease was gradually significant over time, indicating that the NMOSD model was successfully established by injecting the AQP4 antibody from clinically diagnosed patients and the complement into the lateral ventricle of the mice in a manner of stereotactic localization of the brain. The number of AQP4-positive cells in the test article group was significantly higher than that in the model group at days 7-21, and the difference was statistically significant at day 21 (P < 0.001), suggesting that 15 mg/kg mitoxantrone hydrochloride liposome can significantly inhibit the decrease or absent of AQP4 in NMOSD in mechanism and effectively ameliorate the condition of NMOSD in mice.

**Table 2**

| Time point | Group | Mean±SEM | P value |
|---|---|---|---|
| Day 7 | Blank group | 62.0333±2.33405* | 0.015 |
| | Model group | 40.3±6.33745 | / |
| | Test article group | 45.8333±4.10866 | 0.424 |
| Day 11 | Blank group | 64.4667±0.44845** | 0.001 |
| | Model group | 25.7±6.58357 | / |
| | Test article group | 36.5333±4.19100 | 0.141 |
| Day 21 | Blank group | 62.9±0.47258*** | 0.000 |
| | Model group | 5.1±0.5 | / |
| | Test article group | 22.6333±2.5458*** | 0.000 |

| | | | |
|---|---|---|---|
| Note: Mean±SEM, n = 3; Compared to model group: *p<0.05, **p<0.01, ***p<0.001. | | | |

The experimental results showed that mitoxantrone hydrochloride liposome injection (15 mg/kg) can significantly inhibit the decrease or absent of AQP4 in mice, and improve histopathological changes such as astrocytopathology and demyelination, indicating that mitoxantrone hydrochloride liposome injection can effectively improve the condition of NMOSD in mice, and inhibit the occurrence and development thereof.

### Example 2: Phase II clinical study on the efficacy and safety of mitoxantrone hydrochloride liposome injection in the treatment of neuromyelitis optica spectrum disorders (NMOSD)

This example was a randomized, double-blind, and placebo-controlled phase II study. The included subjects will receive the treatment of mitoxantrone hydrochloride liposome injection. The study is aimed to evaluate the safety and efficacy of mitoxantrone hydrochloride liposome injection in the subjects with NMOSDs, and to explore reasonable doses of mitoxantrone hydrochloride liposome injection in the treatment of neuromyelitis optica spectrum disorders (NMOSD), thereby providing basis for later clinical development.

### I. Experimental design

### 1. Experimental procedure

The entire study period is approximately 60 weeks, including a screening period (up to 4 weeks), a treatment period (48 weeks), and a safety follow-up period (8 weeks). The researchers or designees will contact all subjects by telephone every 2 weeks after the start of the treatment period until the experiments are ended up by the subjects (except when the time coincides with the study visit week). If the researchers suspect an onset or imminent onset of NMOSD, an assessment visit will be scheduled within 72 hours.

Subjects diagnosed with NMOSD will be screened within 4 weeks (28 days) to determine their eligibility to participate in the study according to the inclusion and exclusion criteria. The serum status of AQP4-IgG will be determined by the central laboratory. All subjects who met the eligibility criteria were then randomly assigned to the research center. Randomization (Day 1): Eligible subjects will be randomly assigned to experimental group A (mitoxantrone hydrochloride liposome injection, 8 mg/m²), experimental group B (mitoxantrone hydrochloride liposome injection, 12 mg/m²) and placebo group at a ratio of 1:1:1. Randomized subjects will receive the treatment of mitoxantrone hydrochloride liposome or placebo (once every 12 weeks). At 48 weeks after random grouping of the last enrolled patient or NMOSD relapse in the last enrolled patient after random grouping, the treatment period ends up and the safety follow-up period is entered. Safety follow-up will begin after the subjects terminate the trial early or relapse or complete the trials in the treatment period. 8-week safety follow-up (scale evaluation, laboratory examination and electrocardiogram examination, etc.) is required to monitor the subjects for adverse events/serious adverse events and to collect information on the concomitant medications or treatments associated with NMO/NMOSD. During this period, the subjects may receive standard treatment for NMO/NMOSD at the discretion of the researchers.

### 2. Study duration

The study included a screening period of 4 weeks, a treatment period of up to 48 weeks, a safety follow-up period of 8 weeks. It was estimated that the duration of each patient was about 60 weeks (excluding patients with relapse).

45 subjects were scheduled to be enrolled in the study, and the entire period of this study was expected to be 24 months.

### II. Study population

The subjects who met all of the following inclusion criteria and did not have any of the exclusion criteria were enrolled in this clinical study.

### (I) Inclusion criteria

Subjects must meet all of the following criteria:
1) 18-60 years of age (including 18 year old and 60 year old) at the time of signing informed consent, both men and women being eligible;
2) patients who met the International consensus diagnostic criteria for neuromyelitis optica spectrum disorders (NMOSD) in 2015, and who were positive or negative for AQP4-IgG (test results within 24 weeks before signing informed consent being acceptable);
3) clinical evidences of at least 2 episodes within 2 years prior to the screening, and/or of at least 1 documented relapse (including first episode) within 12 months prior to the screening;
4) EDSS≤ 7.5 at the time of screening and baseline retest;
5) voluntarily signing informed consent and voluntarily completing the trial according to protocol requirements

### (II) Exclusion criteria

### 1. Exclusion of general safety

1) Pregnant or lactating women were excluded; for patients of childbearing potential, the serum pregnancy test results were positive at the time of screening or they were unwilling to take reliable contraceptive measures (physical barriers [patients or partners] and spermicide, contraceptive pills, patches, injections, intrauterine devices, or intrauterine systems) and continue for at least 4 months after the last dose of study drug; for men of childbearing potential, effective contraceptive measures were took from the signing of informed consent to 6 months after the last administration.
2) There were evidence of any other demyelinating disease or progressive multifocal leukoencephalopathy (PML) after receiving any surgical operation within 4 weeks prior to randomization.
3) There were active infections (excluding onychomycosis or dental caries) within 4 weeks prior to randomization.
4) Hepatitis B surface antigen (HBsAg), hepatitis C antibody (HCVAb), syphilis antibody, or human immunodeficiency virus antibody (HIV antibody) were positive in the screening of infectious diseases during the screening period.
5) Persons had a history of drug or alcohol abuse (i.e., drinking more than 14 standard units of alcohol per week (1 unit =360 ml beer or 45 ml spirits with 40% alcohol content or 150 ml wine)) or mental disorders within 1 year prior to randomization.
6) There were evidence of active tuberculosis (TB; excluding patients receiving drugs to prevent latent TB infection).
7) There were evidence of active interstitial lung disease.
8) Persons received any live vaccine or attenuated live vaccine within 6 weeks prior to randomization.
9) Persons had a history of malignancies within the past 5 years, including solid tumors, hematological malignancies, and carcinoma in situ (except for skin basal cell carcinoma and squamous cell carcinoma or carcinoma in situ of cervix, which had been completely resected and cured).
10) Persons had a history of severe drug allergy, or were allergic or intolerant to mitoxantrone and liposome (shock, or allergic reaction).
11) Patients had chronic active immune system diseases other than NMOSD or stable diseases but requiring high doses (determined by the researchers) of glucocorticoid to maintain therapy, such as rheumatoid arthritis, scleroderma, Shogren's syndrome, ulcerative colitis, genetic immunodeficiency, or drug-induced immunodeficiency; and patients with only positive autoantibodies but no clinical signs can be enrolled in the trial.
12) At the discretion of the researchers, the persons had other diseases that were not suitable for participation in the study.

### 2. Exclusion criteria associated with prior or concomitant therapy:

13) persons who had received treatment of any research formulation or participated in clinical research of medical devices and might have an impact on the results of this trial according to the judgment of the researchers in the first 3 months prior to randomization;

14) any of the following present in the history of prior treatment for NMOSD:
a. persons who previously received treatment with mitoxantrone or anthracycline, systemic irradiation or bone marrow transplantation at any time;
b. persons who received rituximab or any experimental B cell depletion agent within 6 months prior to randomization, unless the central laboratory considered that the subject had a B cell count higher than LLN;
c. persons who used of any of the following within the first 3 months prior to randomization: satralizumab, tocilizumab, inebilizumab, ecuzumab, alemtuzumab, cyclosporin, azathioprine, methotrexate, cyclophosphamide, tacrolimus, and mycophenolate mofetil; and received any other treatment for preventing recurrence of multiple sclerosis (MS) (e.g., interferon, natalizumab, glatiramer acetate, gilenya, teriflunomide, or dimethyl fumarate);
d. persons who received intravenous immunoglobulin (IVIG) therapy, or plasma exchange therapy (PE) within 1 month prior to randomization;
e. patients who were receiving oral corticosteroid therapy with a dosage higher than 30 mg/ day during conversion of prednisolone;
f. persons who used of anti-CD4 or cladribine within 2 years prior to randomization.

### 3. Exclusion criteria associated with laboratory examination:

15) The following clinically significant diseases were present:
a. cardiac ejection fraction (EF) of below 50% or below the lower limit of detection value by the research central laboratory using echocardiography; a history of chronic congestive heart failure, and NYHA grade III-IV of cardiac function;
b. occurrence of any of the following events within 3 months prior to randomization: myocardial infarction, acute coronary syndrome, viral myocarditis, pulmonary embolism, and stroke; patients undergoing coronary revascularization within 6 months; or
c. QTc interval >480ms (according to the Fridericia correction formula, where QTc=QT/RR^0.33) as indicated by ECG examination during the screening, or patients with a history of severe QTc interval prolongation.

16) The following abnormal laboratory examination values during the screening were present:
a. aspartate aminotransferase (AST) > 3× upper limit of normal (ULN), alanine aminotransferase (ALT) > 3×ULN, total bilirubin > 1.5×ULN (unless due to Gilbert's syndrome);
b. platelet count < 50, 000/µL (or <50 × 10⁹/L), hemoglobin <9 g/dL (or < 90 g/L), leukocyte <2.0 × 10³/µL, absolute neutrophil count <1.0 × 10³/µL; or
c. creatinine clearance (CLcr) < 60 mL/min (calculated by the Cockcroft-Gault formula: [140-age(years)] × [body weight (kg)]×(0.85, if female)/[72×serum creatinine (mg/dL)]), or dialysis during the screening.

### (III)Withdrawal/termination criteria

Subjects can decide to prematurely discontinue the study treatment at any time during the study and this will not affect their continued access to appropriate clinical treatment. Subjects should return all study drugs. The decision of the subject to withdraw from treatment must be recorded in the source file. After withdrawal of informed consent, the patients will not be followed up for any reason. Patients who withdrew from the study will not be replaced.

If one of the following circumstances occurred, the researchers should arrange the subjects to prematurely terminate the study treatment, and follow-up for 8 weeks after termination of treatment and complete the end of study (EOS) visit assessment as far as possible.
1) The subjects requested withdrawal from the trial or the subjects were lost to follow-up.
2) The subjects were pregnant.
3) The researchers or the sponsors decided that the subjects should withdraw from the treatment for safety reasons when a serious adverse event or an adverse event with greater risk hidden danger occurred in the subjects.
4) Any of the following abnormal liver functions was present in the subjects:
   a. ALT or AST > 8×ULN
   b. ALT or AST > 5×ULN for more than 2 weeks (no bilirubin increase and/or presence of fatigue, nausea, vomiting, pain or tenderness in right upper abdominal, fever, rash and/or eosinophilia (> 5%);
   c. ALT or AST > 3×ULN and total bilirubin > 2×ULN or international normalized ratio [INR]> 1.5, no cholestasis factor found; or
   d. ALT or AST > 3×ULN with fatigue, nausea, vomiting, pain or tenderness in right upper abdominal, fever, rash and/or eosinophilia (> 5%).
5) During the study, subjects diagnosed with NMOSD relapse according to criteria defined in the protocol, or those with cardiotoxicity (LVEF below the lower limit or clinically significant decrease in LVEF) are required to discontinue study treatment and to withdraw from the study upon judgement by the researchers.
6) If the subjects delayed administration for more than 14 days or experienced a major violation of the protocol, the researchers judged that the subjects should withdraw from the treatment for the consideration of compliance or benefit.
7) Other circumstances are present, in which the researchers considered the patients unsuitable to continue to participate in the trial.

### III. Efficacy evaluation index

### Primary endpoint: time to first relapse

The time to first relapse refers to the time from randomization of the subjects to the first onset of NMOSD. NMOSD relapse is defined as the occurrence of new symptoms or worsening of existing symptoms associated with NMOSD, which meet at least one of criteria of the NMOSD onset of the definition of relapse in the protocol.

### Secondary Endpoints

- Annualized relapse rate (ARR): The ARR at week 48 was calculated as the ratio of the sum of times of confirmed relapses in all participants divided by the sum of treatment time (in years) of all participants.
- Change of expanded disability status scale (EDSS) score relative to baseline
- Change of modified Rankin Scale (mRS) score relative to baseline
- Change of T25FW score relative to baseline
- Change of EQ-5D score relative to baseline
- Change of low contrast visual acuity (LCVA) score relative to baseline
- Change of pain NRS score at 5 sites relative to baseline
- Change of serum glial fibrillary acidic protein (sGFAP) and serum neurofilament light chain protein NfL levels relative to baseline
- Serum AQP4 titer

### IV. Research Results

For patients with neuromyelitis optica spectrum disorders, treatment with mitoxantrone hydrochloride liposome injection was safe and tolerable, had little toxic and side effects, improved treatment effectiveness, and delayed recurrence of neuromyelitis optica spectrum disorders.

The implementations of the technical solutions of the present application have been illustratively described above. It is to be understood that the protection scope of the present application should not be unduly limited to these specific embodiments. Any modifications, equivalents, and improvements, etc. made by those skilled in the art within the spirit and principles of this application are intended to be included within the protection scope of the claims of this application.

## Claims

1. Use of mitoxantrone hydrochloride liposome in the manufacture of a medicament for treating neuromyelitis optica spectrum disorders, wherein preferably, the neuromyelitis optica spectrum disorders are highly recurrent neuromyelitis optica spectrum disorders, and more preferably, the highly recurrent neuromyelitis optica spectrum disorders are those for which the treatment with other drugs failed.

2. A method for treating neuromyelitis optica spectrum disorders in an individual, comprising administering to the individual a therapeutically effective amount of mitoxantrone hydrochloride liposome or a pharmaceutical composition comprising mitoxantrone hydrochloride liposome, wherein preferably, the neuromyelitis optica spectrum disorders are highly recurrent neuromyelitis optica spectrum disorders, and more preferably, the highly recurrent neuromyelitis optica spectrum disorders are those for which the treatment with other drugs failed.

3. Mitoxantrone hydrochloride liposome or a pharmaceutical composition comprising mitoxantrone hydrochloride liposome for use in treating neuromyelitis optica spectrum disorders, wherein preferably, the neuromyelitis optica spectrum disorders are highly recurrent neuromyelitis optica spectrum disorders, and more preferably, the highly recurrent neuromyelitis optica spectrum disorders are those for which the treatment with other drugs failed.

4. The use according to claim 1, the method according to claim 2, or the mitoxantrone hydrochloride liposome or the pharmaceutical composition comprising mitoxantrone hydrochloride liposome according to claim 3, wherein the mitoxantrone hydrochloride liposome is used as the sole active ingredient for the treatment of neuromyelitis optica spectrum disorders, or the mitoxantrone hydrochloride liposome is used alone for the treatment of neuromyelitis optica spectrum disorders.

5. The use according to claim 1, the method according to claim 2, or the mitoxantrone hydrochloride liposome or the pharmaceutical composition comprising mitoxantrone hydrochloride liposome according to claim 3, wherein the medicament or pharmaceutical composition is an injectable formulation, preferably a liquid injection, a powder for injection, or a tablet for injection; and more preferably, the medicament or pharmaceutical composition is a liquid injection.

6. The use according to claim 1, the method according to claim 2, or the mitoxantrone hydrochloride liposome or the pharmaceutical composition comprising mitoxantrone hydrochloride liposome according to claim 3, wherein calculated as mitoxantrone, the content of the active ingredient in the medicament or pharmaceutical composition is 0.5-5 mg/ml, preferably 1-2 mg/ml, and more preferably 1 mg/ml.

7. The use according to claim 1, the method according to claim 2, or the mitoxantrone hydrochloride liposome or the pharmaceutical composition comprising mitoxantrone hydrochloride liposome according to claim 3, wherein the mitoxantrone hydrochloride liposome has a particle size of about 30-80 nm, for example about 35-75 nm, preferably about 40-70 nm, more preferably about 40-60 nm, for example about 60 nm.

8. The use according to claim 1, the method according to claim 2, or the mitoxantrone hydrochloride liposome or the pharmaceutical composition comprising mitoxantrone hydrochloride liposome according to claim 3, wherein the active ingredient mitoxantrone in the mitoxantrone hydrochloride liposome forms a poorly soluble precipitate with multivalent counterions in the liposome, wherein the multivalent counterions are, for example, sulfate radicals, citrate radicals or phosphate radicals.

9. The use according to claim 1, the method according to claim 2, or the mitoxantrone hydrochloride liposome or the pharmaceutical composition comprising mitoxantrone hydrochloride liposome according to claim 3, wherein the phospholipid bilayer in the mitoxantrone hydrochloride liposome comprises phospholipids with a phase transition temperature (Tm) higher than body temperature, such that the phase transition temperature of the liposome is higher than body temperature, and for example, the phospholipids are selected from the group consisting of hydrogenated soybean lecithin, phosphatidylcholine, hydrogenated egg yolk lecithin, bispalmitate lecithin, bisstearate lecithin, or any combination thereof.

10. The use according to claim 1, the method according to claim 2, or the mitoxantrone hydrochloride liposome or the pharmaceutical composition comprising mitoxantrone hydrochloride liposome according to claim 3, wherein the mass ratio of hydrogenated soybean lecithin, cholesterol and polyethylene glycol 2000 modified distearoyl phosphatidylethanolamines is 3:1:1; and/or
preferably, wherein the weight ratio of hydrogenated soybean lecithin, cholesterol, polyethylene glycol 2000 modified distearoyl phosphatidylethanolamines and mitoxantrone is 9.58:3.19:3.19:1; and/or
preferably, wherein the particle size is about 60 nm; and/or
preferably, wherein the counterions are sulfate radical ions.

11. The method according to claim 2, wherein calculated as mitoxantrone, the therapeutically effective amount is 4-30 mg/m², preferably 8-20 mg/m² or 12-30 mg/m², for example 8 mg/m² or 12 mg/m²; and/or
the mode of administration is intravenous administration; and/or
the dosing period is once every 12 weeks.
